# EUROPEAN PATENT SPECIFICATION

(11) **EP 2 269 069 B1**
(45) Date of publication and mention of the grant of the patent: **26.10.2011**
(21) Application number: 09719883.2
(22) Date of filing: 10.03.2009
(51) Int. Cl.: G01N 33/569, C07K 17/00

(54) **USE OF SYNTHETIC PEPTIDE DERIVED FROM ZEBRA PROTEIN FOR THE IN VIVO DIAGNOSIS OF THE EPSTEIN-BARR VIRUS (EBV) REACTIVATION**
VERWENDUNG EINES SYNTHETISCHEN PEPTIDS AUS DEM ZEBRA-PROTEIN FÜR DIE IN-VITRO DIAGNOSE VON EPPSTEIN-BARR-VIRUS (EBV)-REAKTIVIERUNG
UTILISATION DE PEPTIDE SYNTHÉTIQUE DÉRIVÉ DE LA PROTÉINE ZEBRA POUR LE DIAGNOSTIC IN VITRO DE LA RÉACTIVATION DU VIRUS D'EPSTEIN-BARR (EBV)

(30) Priority: 10.03.2008 EP 08290224
(43) Date of publication of application: 05.01.2011
(73) Proprietor: Université Joseph Fourier, 38041 Grenoble Cedex 09 (FR)
(72) Inventor: DROUET, Emmanuel, F-38700 Corenc (FR)
(74) Representative: Mouget-Goniot, Claire
(86) International application number: PCT/EP2009/052798
(87) International publication number: WO 2009/112497

(56) References cited:
- WO-A-00/55622
- WO-A-96/21155
- MARECHAL V ET AL.: "Enzyme-linked immunosorbent assay for antibodies to ZEBRA, an Epstein-Barr trans-activator" RESEARCH VIROLOGY, vol. 144, no. 5, 1993, pages 397-404, XP022352696 cited in the application
- DROUET E ET AL.: "High Epstein-Barr Virus Serum Load and Elevated Titers of Anti-ZEBRA Antibodies in Patients With EBV-Harboring Tumor Cells of Hodgkin's Disease" JOURNAL OF MEDICAL VIROLOGY, vol. 57, 1999, pages 383-389, XP002489204 cited in the application
- TOUGE C ET AL.: "High incidence of elevated antibody titers to Epstein-Barr-virus in patients with uveitis" ARCHIVES OF VIROLOGY, vol. 151, 2006, pages 895-903, XP019378678 cited in the application
- TEDESCHI R ET AL.: "Activation of Maternal Epstein-Barr Virus Infection and Risk of Acute Leukemia in the Offspring" AMERICAN JOURNAL OF EPIDEMIOLOGY, vol. 165, no. 2, 2006, pages 134-137, XP002489206 cited in the application
- DARDARI R ET AL.: "Analyses of the prognostic significance of the Epstein-Barr virus transactivator ZEBRA protein and diagnostic value of its two synthetic peptides in nasopharyngeal carcinoma" JOURNAL OF CLINICAL VIROLOGY, vol. 41, no. 2, February 2008 (2008-02), pages 96-103, XP022503936 cited in the application
- DARDARI R ET AL: "Antibody Responses to Recombinant Epstein-Barr Virus Antigens in Nasopharyngeal Carcinoma Patients: Complementary Test of ZEBRA Protein and Early Antigens p54 and p138" JOURNAL OF CLINICAL MICROBIOLOGY, vol. 39, no. 9, 2001, pages 3164-3170, XP002489208 cited in the application

### SEQUENCE LISTING

<110> University Joseph Fourier DROUET, Emmanuel
<120> USE OF SYNTHETIC PEPTIDE DERIVED FROM ZEBRA PROTEIN FOR THE IN VITRO DIAGNOSIS OF THE EPSTEIN-BARR VIRUS (EBV) REACTIVATION.
<130> WOB 07 CS UJF P125
<150> EP 08 290 224.8
   <151> 2008-03-10
<160> 8
<170> PatentIn version 3.5
<210> 1
   <211> 7
   <212> PRT
   <213> Artificial sequence
<220>
   <223> Xaa = all amino acids, except Pro
<220>
   <221> misc_feature
   <222> (1)..(1)
   <223> Xaa can be any naturally occurring amino acid, except Pro
<220>
   <221> misc_feature
   <222> (3)..(3)
   <223> Xaa can be any naturally occurring amino acid, except Pro
<220>
   <221> misc_feature
   <222> (5)..(5)
   <223> Xaa can be any naturally occurring amino acid, except Pro
<220>
   <221> misc_feature
   <222> (7)..(7)
   <223> Xaa can be any naturally occurring amino acid, except Pro
<400> 1
   Xaa Pro Xaa Pro Xaa Pro Xaa
   1               5
<210> 2
   <211> 19
   <212> PRT
   <213> Artificial sequence
<220>
   <223> derived from Epstein-Barr Virus Zebra protein
<400> 2
   Gly Gln Leu Thr Ala Tyr His Val Ser Thr Ala Pro Thr Gly Ser Trp
   1               5                   10                  15
   Phe Ser Ala
<210> 3
   <211> 12
   <212> PRT
   <213> Artificial sequence
<220>
   <223> derived from Epstein-Barr virus Zebra protein
<400> 3
   Glu Asn Ala Tyr Gln Ala Tyr Ala Pro Gln Leu Phe
   1               5                   10
<210> 4
   <211> 36
   <212> PRT
   <213> Artificial sequence
<220>
   <223> derived from Epstein-Barr virus Zebra protein
<400> 4
   Gly Gln Leu Thr Ala Tyr His Val Ser Thr Ala Pro Thr Gly Ser Trp
   1               5                   10                  15
   Phe Ser Ala Pro Gln Pro Ala Pro Glu Asn Ala Tyr Gln Ala Tyr Ala
               20                  25                  30
   Pro Gln Leu Phe
           35
<210> 5
   <211> 12
   <212> PRT
   <213> Artificial sequence
<220>
   <223> derived from Epstein-Barr Virus Zebra protein
<400> 5
   Phe Ser Ala Pro Gln Pro Ala Pro Glu Asn Ala Tyr
   1               5                   10
<210> 6
   <211> 245
   <212> PRT
   <213> Epstein-Barr Virus
<400> 6
   Met Met Asp Pro Asn Ser Thr Ser Glu Asp Val Lys Phe Thr Pro Asp
   1               5                   10                  15
   Pro Tyr Gln Val Pro Phe Val Gln Ala Phe Asp Gln Ala Thr Arg Val
               20                  25                  30
   Tyr Gln Asp Leu Gly Gly Pro Ser Gln Ala Pro Leu Pro Cys Val Leu
           35                  40                  45
   Trp Pro Val Leu Pro Glu Pro Leu Pro Gln Gly Gln Leu Thr Ala Tyr
       50                  55                  60
   His Val Ser Thr Ala Pro Thr Gly Ser Trp Phe Ser Ala Pro Gln Pro
   65                  70                  75                  80
   Ala Pro Glu Asn Ala Tyr Gln Ala Tyr Ala Ala Pro Gln Leu Phe Pro
                   85                  90                  95
   Val Ser Asp Ile Thr Gln Asn Gln Gln Thr Asn Gln Ala Gly Gly Glu
               100                 105                 110
   Ala Pro Gln Pro Gly Asp Asn Ser Thr Val Gln Thr Ala Ala Ala Val
           115                 120                 125
   Val Phe Ala Cys Pro Gly Ala Asn Gln Gly Gln Gln Leu Ala Asp Ile
       130                 135                 140
   Gly Val Pro Gln Pro Ala Pro Val Ala Ala Pro Ala Arg Arg Thr Arg
   145                 150                 155                 160
   Lys Pro Gln Gln Pro Glu Ser Leu Glu Glu Cys Asp Ser Glu Leu Glu
                   165                 170                 175
   Ile Lys Arg Tyr Lys Asn Arg Val Ala Ser Arg Lys Cys Arg Ala Lys
               180                 185                 190
   Phe Lys Gln Leu Leu Gln His Tyr Arg Glu Val Ala Ala Ala Lys Ser
           195                 200                 205
   Ser Glu Asn Asp Arg Leu Arg Leu Leu Leu Lys Gln Met Cys Pro Ser
       210                 215                 220
   Leu Asp Val Asp Ser Ile Ile Pro Arg Thr Pro Asp Val Leu His Glu
   225                 230                 235                 240
   Asp Leu Leu Asn Phe
                   245
<210> 7
   <211> 221
   <212> PRT
   <213> Artificial Sequence
<220>
   <223> Derived from Epstein-Barr virus Zebra protein (Zebra delta N 24)
<400> 7
   Ala Phe Asp Gln Ala Thr Arg Val Tyr Gln Asp Leu Gly Gly Pro Ser
   1               5                   10                  15
   Gln Ala Pro Leu Pro Cys Val Leu Trp Pro Val Leu Pro Glu Pro Leu
               20                  25                  30
   Pro Gln Gly Gln Leu Thr Ala Tyr His Val Ser Thr Ala Pro Thr Gly
           35                  40                  45
   Ser Trp Phe Ser Ala Pro Gln Pro Ala Pro Glu Asn Ala Tyr Gln Ala
       50                  55                  60
   Tyr Ala Ala Pro Gln Leu Phe Pro Val Ser Asp Ile Thr Gln Asn Gln
   65                  70                  75                  80
   Gln Thr Asn Gln Ala Gly Gly Glu Ala Pro Gln Pro Gly Asp Asn Ser
                   85                  90                  95
   Thr Val Gln Thr Ala Ala Ala Val Val Phe Ala Cys Pro Gly Ala Asn
               100                 105                 110
   Gln Gly Gln Gln Leu Ala Asp Ile Gly Val Pro Gln Pro Ala Pro Val
           115                 120                 125
   Ala Ala Pro Ala Arg Arg Thr Arg Lys Pro Gln Gln Pro Glu Ser Leu
       130                 135                 140
   Glu Glu Cys Asp Ser Glu Leu Glu Ile Lys Arg Tyr Lys Asn Arg Val
   145                 150                 155                 160
   Ala Ser Arg Lys Cys Arg Ala Lys Phe Lys Gln Leu Leu Gln His Tyr
                   165                 170                 175
   Arg Glu Val Ala Ala Ala Lys Ser Ser Glu Asn Asp Arg Leu Arg Leu
               180                 185                 190
   Leu Leu Lys Gln Met Cys Pro Ser Leu Asp Val Asp Ser Ile Ile Pro
           195                 200                 205
   Arg Thr Pro Asp Val Leu His Glu Asp Leu Leu Asn Phe
       210                 215                 220
<210> 8
   <211> 15
   <212> PRT
   <213> Artificial Sequence
<220>
   <223> Derived from Epstein-Barr virus Zebra protein
<400> 8
   Phe Ser Ala Pro Gln Pro Ala Pro Glu Asn Ala Tyr Gly Ser Lys
   1               5                   10                  15

## Description

The present invention relates to the use of a synthetic peptide derived from ZEBRA protein for *the in vitro* diagnosis of the Epstein-Barr virus (EBV) reactivation. The present invention particularly relates to a method for the determination of the level of IgG antibodies for the diagnosis of EBV reactivation.

Epstein-Barr virus (EBV) is a gamma-herpes virus establishing a latent infection in human B lymphocytes, efficiently transforming the cells into lymphoblastoid cell lines (LCLs), and is implicated in the etiology of infectious mononucleosis, Burkitt's lymphoma (BL), Hodgkin's disease, nasopharyngeal carcinoma (NPC) and lymphoproliferative diseases in immunocompromised individuals (Epstein MA & Crawford DH, 2005; Klein G, 2005; Cohen JI, 2005).
While primary infection is often accompanied by a self-limited period of clinical illness, long-term latency is symptom-free. Following activation, transcription of viral genes switches from latent to *lytic* phase that leads to enhanced replication and virus production. The virus is capable of adopting four programs of latency (latency 0, I, II and III).
In healthy individuals, latent EBV infection appears to be primarily confined to resting memory B cells (Babcock *et al.,* 1998). The only EBV gene products that are consistently detected in these cells is (i) the latent membrane proteins LMP1 and LMP2A/2B, (ii) the EBNAs (Epstein-Barr Nuclear Antigens), defining a pattern of gene expression at present termed latency (Thorley-Lawson *et al.,* 1996).
In Burkitt's lymphoma biopsies, only Epstein-Barr virus nuclear antigen 1 (EBNA1) is expressed (latency I). In Hodgkin's disease, NPC and T cell lymphomas, EBNA1 and variable combinations of the three members of the latent membrane protein family (LMP1, LMP2A and LMP2B) are expressed (latency II). During acute infectious mononucleosis, in lymphoproliferative syndromes in immunocompromised individuals and in LCLs established *in vitro,* all six nuclear antigens (EBNA1-6) are expressed (latency III). In addition, all three LMPs are expressed. EBV-infected cell lines are either completely non-productive for virus particles or else contain a small subpopulation of cells that have switched spontaneously from a latent stage of infection into the lytic cycle.
The mechanism of switching is not fully understood, but one of the first detectable changes in viral gene expression is activation of the EBV immediate-early gene BZLF1, which encodes the lytic switch transactivator ZEBRA (Flemington *et al.,* 1991). ZEBRA, together with the protein product of the BRLF1 gene, then initiates the lytic cycle cascade (Feederle et al. 2000).

EBV-positive adults, immunologically healthy or immunosuppressed, present the same level of EBV virus lytic replication (Hong et al., 2005; Montone et al, 1996). This active replication of the virus is probably required for the lifelong persistence.

Whereas immmunocompetent individuals can limit proliferation of EBV-infected cells and exhibit often standardized serologic profile, those with congenital or acquired immunodeficiency are highly susceptible to EBV-associated lymphoproliferations.

ZEBRA protein (SEQ ID NO 6) is a transcription factor and contains three functional domains consisting of the amino-terminal part of a transactivator domain, a DNA binding domain and in the carboxy-terminal part of the protein, a dimerization domain.
ZEBRA is a highly immunogenic protein and contain many epitopes liable to be recognized by antibodies.
Some studies disclose the use of ZEBRA protein to detect EBV reactivation:
Drouet et al (Drouet et al. 1999) disclose the use of ZEBRA protein for the Hodgkin disease detection. ZEBRA allows to detect in patient's serum antibodies directed against ZEBRA, in a dose dependant manner.
Touge et al. (Touge et al., 2006) and Tedeschi et al. (Tedeschi et al., 2006) disclose the use oz ZEBRA in order to detect EBV reactivation in patients afflicted by uveitis or leukemia, respectively.
Dardari et al. (Dardari et al., 2001) disclose the use of ZEBRA and p54 and p138 proteins in order to enhance the EBV reactivation.
Although all the previous documents disclose methods using ZEBRA protein for detecting EBV reactivation, none of these documents mentions that fragments of ZEBRA can be used for providing a method for detecting EBV reactivation.

Among the ZEBRA epitopes, two major polypeptides containing the major immunogenic regions of ZEBRA, P130 ZEBRA and P125 ZEBRA, have been identified. P130 ZEBRA corresponds to the amino acids 157 to 195 of the ZEBRA protein, and P125 corresponds to amino acids 59 to 93 of the ZEBRA protein.

Among the antibodies produced during the course of infection, antibodies raised against ZEBRA Protein are detected during a particular process called EBV reactivation (Maréchal et al. 1993, Joab et al. 1991, Brousset et al. AIDS 1994).

Serology has been an important aid in the diagnosis of EBV infections well before the discovery of the virus itself in 1964. Infection with EBV produces a wide range of antibodies to the various antigens, both structural and non structural (Henle W and Henle G, 1981).
Over the course of the infection, the levels of antibodies to the different EBV antigens will rise and then decrease, providing valuable information on the stage of the infection. Individual differences in the immune system of patients mean that no people will produce exactly the same antibody profile at a given time. However, the sequence of antibody production during the course of the infection is consistent enough to provide useful diagnostic information.

The apparition of anti-ZEBRA antibodies is an important event during the serologic diagnosis of pathologies associated with EBV and during the follow-up of patients with susceptibility to develop a pathology associated with EBV (immunosuppressed patients and patients afflicted by neoplasia associated to EBV infection).

Some works have described P130 and P125, and the use of their immunogenic properties, as diagnosis and prognosis markers for EBV infection detection and during progression of pathologies associated with EBV infection.

WO96/21155 discloses a method for determining the presence or absence of P130 ZEBRA protein during a primary EBV infection. This document only recites the use of P130 during the first steps of the disease, but not during the second rebound of the illness, e.g. EBV reactivation.

Dardari et al. (Dardari et al. 2007) describes prognosis significance of P125 and P130 ZEBRA proteins during Nasopharyngeal carcinoma (NPC). This article demonstrates that antibodies directed against P125 are more present in patients with NPC, whatever their age, than antibodies directed against P 130. However, this article does not describe the use of other EBV proteins as prognosis marker.

WO00/55622 recites a pharmaceutical composition comprising, in particular P100 ZEBRA protein, to prevent infection by EBV virus, and to prevent the development of pathologies such as Burkitt's lymphoma, Hodgkin's disease and Nasopharyngeal carcinoma.

Marechal et al. (Research Virology, 1993, vol 144, n°5,p 397-404**) recite an ELISA test comprising ZEBRA for screening EBV reactivation in HIV positive patients.**

There is no method, to date, that allows efficiently and rapidly the detection of EBV reactivation in healthy patient infected with EBV virus or patients afflicted with pathologies associated with EBV infection.

The invention is based on the unexpected observation that IgG antibodies against P100 ZEBRA protein appear more rapidly than antibodies against P 130 or any other protein from EBV virus, such as EBNA, VCA and EA proteins. In particular, the inventors have surprisingly discovered that P100 ZEBRA polypeptide is a very efficient immunogenic polypeptide able to be used to detect antibodies produced during the EBV reactivation.

The aim of the invention is to provide a method for detecting IgG antibodies against EBV ZEBRA protein during pathologies associated with EBV reactivation in patient.

The present disclosure relates to the use of at least one polypeptide derived from ZEBRA protein comprising at least the following amino acid sequence represented by SEQ ID NO 1, for the *in vitro* and *ex vivo* screening of the EBV virus reactivation in a biological sample of a subject afflicted by a pathology associated with EBV infection.
The used polypeptide, represented by SEQ ID NO 1, is defined by the following sequence -X1-P-X2-P-X3-P-X4-, wherein:
- X1 can represent an amino acid, with the exception of Proline, or a sequence from any two consecutive amino acids to any nineteen consecutive amino acids, wherein the last amino acid is not a Proline,
- X2 and X3 can represent independently from each other one amino acid selected from the known amino acids, with the exception of Proline,
- X4 can represent an amino acid, with the exception of Proline, or a sequence from any two consecutive amino acids to any twelve consecutive amino acids, wherein the first amino acid is not a Proline.

The invention relates to the use of the ZEBRA P100 fragment consisting of the sequence SEQ ID NO 5 or SEQ ID NO 8,
for the *in vitro* and ex *vivo* screening of the EBV virus reactivation in a biological sample of a subject afflicted by a pathology associated with EBV infection.

According to the disclosure, amino acids are chosen among the group consisting in the twenty natural amino acids: Alanine, Arginine, Asparagine, Aspartic acid, Cysteine, Glutamic acid, Glycine, Histidine, Isoleucine, Leucine, Lysine, Methionine, Proline, Serine, Tryptophane, Tyrosine and Valine. According to the invention, each amino acid can also be chosen among the non standard amino acids: Selenocysteine, Pyrrolysine, lanthionine, 2-aminoisobutyric acid, dehydroalanine, gamma-aminobutyric acid., ornithine, and citrulline. Nonstandard amino acids are usually formed through modifications to standard amino acids. According to the invention amino acid can also correspond to homocysteine S-adenosyl methionine and hydroxyproline.

The disclosure also relates to the uses of variants or isoformes of at least one polypeptide characterized in that it comprises amino acid sequence represented by SEQ ID NO 1.
The invention does not concern to the use of this polypeptide for the *in vitro* and *ex vivo* screening of the EBV virus reactivation in a biological sample of a subject when the said polypeptide corresponds to the amino acids sequence SEQ ID NO 4, SEQ ID NO 6 and SEQ ID NO 7 corresponding to SEQ ID NO 6 in which 24 amino acids in the N-terminus have been deleted.

One embodiment of the disclosure relates to the use of a polypeptide derived from ZEBRA protein comprising at least the following amino acid sequence : -X1-P-X2-P-X3-P-X4- in which:
X1 is -A1-A2-A3, A1 being chosen among F, Y, W, A2 among S, T, Y, and A3 among G, A, V, L, I,
X2 is chosen among Q, N, E, D,
X3 is chosen among G, A, V, L, I,
X4 is-B1-B2-B3-B4-, B1 being chosen among E, D, N, Q, B2 among N, Q, D, E, B3 among G, A, V, L, I, et B4 among F, Y, W, X1 X2, X3,
for the *in vitro* and *ex vivo* screening of the EBV virus reactivation in a biological sample of a subject afflicted by a pathology associated with EBV infection.

The polypeptide corresponding to the P125 ZEBRA polypeptide is represented by SEQ ID NO 4. P125 polypeptide is contained in ZEBRA protein, and corresponds to the amino acids 59-93 of ZEBRA protein.
When the polypeptide SEQ ID NO 1 is P125 ZEBRA polypeptide, X1, X2, X3 and X4 are defined such as:
X1: GQLTAYHVSTAPTGSWFSA, represented SEQ ID NO 2, and
X4: ENAYQAYAPQLF, represented by SEQ ID NO 3,
X2: Q, and
X3: A.

In the context of the invention, the polypeptide -X1-P-X2-P-X3-P-X4- corresponds to the P100 ZEBRA polypeptide, represented by SEQ ID NO 5. P100 ZEBRA polypeptide corresponds to the amino acids 75-86 of ZEBRA protein. Therefore, the amino acid sequence of the polypeptide used is -F-S-A-P-Q-P-A-P-E-N-A-Y-.

According to the disclosure, a polypeptide derived from ZEBRA protein comprising at least the following amino acid sequence represented by SEQ ID NO 1 can be also modified in order to graft a molecule or a compound that allows the detection, or the anchoring, of said polypeptide derived from ZEBRA protein.
For instance, such molecule or compound can be biotin, streptavidin, protein tags, fluorescent molecule, said molecule or a compound being commonly used by a skilled person.
Thus, another specific polypeptide for use according to the invention is represented by the amino acid sequence SEQ ID NO 8 (-F-S-A-P-Q-P-A-P-E-N-A-Y-G-S-K-) corresponding to P100 polypeptide (SEQ ID NO 5) in which G-S-K peptide is added at the C-terminus end of P100 sequence.

According to the disclosure, the terms "polypeptides" and "peptides" mean fragment of a protein, constituted by at least two amino acids. By extension, "polypeptide of ZEBRA protein" means a fragment of the ZEBRA protein, constituted by at least SEQ ID NO 1.

According to the disclosure, "variant" is defined as a polypeptide that differs from the reference polypeptide, but retains essential properties. Variants and reference polypeptide share similar amino acids sequences with, for example, 90% of amino acids identity, preferably 95% of amino acids identity, and more preferably 99% of amino acids identity. By "isoform", it is defined according to the description a polypeptide that differs from the reference polypeptide with essential conserved properties, said isoforms being coded by products of genes that result of an alternative splicing of a same gene or by products resulting of the expression of several homologous genes of which sequences have diverged.

The use of the disclosure permits to determine the presence or absence of IgG antibodies directed against ZEBRA polypeptide described above, in a biological sample from a subject.

In one embodiment, the disclosure also relates to the use of at least one polypeptide derived from ZEBRA protein for the *in vitro* and *ex vivo* screening of the EBV virus reactivation in a biological sample of a subject afflicted by a pathology associated with EBV infection, wherein pathologies associated with EBV infections are selected from the group comprising: tumors specific to the immunocompromised host, tumor of the immunocompetent host and viral syndrome related to EBV.
The biological sample of the invention is a body fluid, preferably blood or plasma. The body fluid could be eventually saliva, urine or lymph. Any other body fluid could be considered in the invention.

According to the invention, the pathologies associated with EBV infection are defined as pathologies during which the EBV virus is present in the virus host cells.
Three different steps represent EBV virus infection: primo-infection, latency step and reactivation. According to the invention, pathologies associated with EBV infection preferably describe virus reactivation, but do not exclude latency step. However, the invention does not cover the pathologies corresponding to the EBV virus primo-infection.
The subject afflicted by the pathology associated with EBV infection develops characteristic symptoms of the illness, well known by the skilled man in the art. The characteristic symptoms of the illness are determined by the physiopathological and clinical knowledge of the illness (Henle W and Henle G, 1981).
In the invention, the terms "pathology", "illness", "disease" and "malignancy" are used uniformly to define an abnormal condition of an organism that impairs bodily functions.

The pathologies described in the present invention are related to the EBV infection. More particularly, they concern pathologies associated with immune system disorders, leading to susceptibility to opportunistic infections
- Tumors specific to the immunocompromised host defined in the invention comprise B-cell lymphoproliferative diseases and smooth muscles cells tumors.
- Tumors of the immunocompetent host, defined in the invention, comprise pathologies associated with the infection of both mononuclear cells and epithelial cells, such as Hodgkin's lymphoma and Burkitt lymphoma, and non B-cells, such as T-cells and NK-cells lymphoma and Nasopharyngeal carcinoma (NPC).

The invention is also based on the observation made by the inventors regarding a method for *in vitro* and *ex vivo* determination of the presence or amount of at least one antibody that specifically recognizes polypeptide -X1-P-X2-P-X3-P-X4- (SEQ ID NO 1), or variants or isoforms of the said polypeptide, wherein
- X1 can represent an amino acid, with the exception of Proline, or a sequence from any two consecutive amino acids to any nineteen consecutive amino acids, wherein the last amino acid is not a Proline,
- X2 and X3 can represent independently from each other one amino acid selected from the known amino acids, with the exception of Proline,
- X4 can represent an amino acid, with the exception of Proline, or a sequence from any two consecutive amino acids to any twelve consecutive amino acids, wherein the first amino acid is not a Proline,
said antibody being liable to be present in a and biological sample of a subject afflicted by a pathology associated with EBV infection.
According to the invention, the method does not concern the *in vitro* and *ex vivo* determination of the presence or amount of at least one antibody that specifically recognizes P125 ZEBRA polypeptide (SEQ ID NO 4), or polypeptides SEQ ID NO 6 or SEQ ID NO 7. In a preferred embodiment, it is disclosed a method for *in vitro* and *ex vivo* determination of the presence or amount of at least one antibody described above, wherein said antibody specifically recognizes polypeptide SEQ ID NO 1 in which:
X1 is -A1-A2-A3, A1 being chosen among F, Y, W, A2 among S, T, Y, and A3 among G, A, V, L, I,
X2 is chosen among Q, N, E, D,
X3 is chosen among G, A, V, L, I,
X4 is-B1-B2-B3 B4-, B1 being chosen among E, D, N, Q, B2 among N, Q, D, E,
B3 among G, A, V, L, I, et B4 among F, Y, W, X1, X2, X3.

The invention also relates to a method for *in vitro* and *ex vivo* determination of the presence or amount of at least one antibody that specifically recognizes the polypeptide consisting of SEO ID NO 5 or SEO ID NO 8,
said antibody being liable to be present in a biological sample of a subject afflicted by a pathology associated with EBV infection.

In a preferred embodiment, the invention discloses a method for *in vitro* and *ex vivo* determination of the presence or amount of at least one antibody described above, wherein said antibody specifically recognizes polypeptides SEQ ID NO 5, corresponding to the P100 ZEBRA polypeptide.

According to the invention, the determination of the presence of at least one antibody indicates that if an antibody can be detected in a biological sample, the antibody is considered as present in the biological sample. On the contrary, if the said antibody can not be detected by the method of the invention, the antibody is considered as absent from the biological sample.
By antibody, it is defined in the invention all the immunological molecules produced by B-cell: immunoglobulins (Ig). Then, according to the invention, all the soluble and insoluble immunoglobulins, such as IgG, IgM, IgA and IgD can be detected. According to the invention IgG antibodies are preferably detected.
With regard to the determination of the quantification of amount of at least an antibody, it is heard in the invention, that the quantity of said antibody is measured.
The amount of antibody is measured using a classical protocol of quantification, wherein the amount of antibody is compared with at least two control samples. These control samples are represented by at least a negative sample and a positive control sample. The value associated to the measure of the quantity of antibody is null in the control negative sample, and value associated to the measure of the quantity of antibody is positive in the control positive sample. So, if the antibody is absent of the biologic sample, the value of the quantification is null. On the other hand, if the antibody is present, the value of the quantification is superior to zero. The presence or amount of antibodies may be determined by any routine protocols commonly used in the art.

According to the method of the disclosure, polypeptides are recognized specifically by at least one antibody liable to be present in a biological sample of a subject. When the antibody is present, the recognition is said specific, which means that the antibody only interact with said polypeptide, or the variants or isoforms of the polypeptides, but does not interact with another polypeptide.
The disclosure describes a method that allows to detect, in a biologic sample, an IgG antibody that specifically recognize the peptide consisting in sequence SEQ ID NO 1.
The invention relates to a method for the detection, in a biological sample, of antibodies that specifically recognize polypeptides corresponding to SEQ ID NO 5, or SEQ ID NO 8.

The method disclosed in the invention comprises, in a specific embodiment, at least two steps:
a. contacting a biological sample from a patient with at least one polypeptide, preferably a polypeptide chosen among the group consisting of SEQ ID NO 8 and SEQ ID NO 5,
b. detecting the formation of an immune complex between said polypeptide and antibody liable to be present in the said biological sample.
Optionally, a supplemental step can be added to the method. This additional step allows quantifying the amount of formed immune complex, by comparing the amount of formed immune complex with the known amount of formed immune complex of control samples. This quantification can be performed according a routine protocol, for example comparing value of formed immune complex with the values of known immune complexes, said values of known immune complexes defining a standard curve.
The method according to the invention does not concern the in vitro and ex vivo determination of the presence or amount of at least one antibody that specifically recognizes P125 ZEBRA polypeptide (SEQ ID NO 4), or polypeptides SEQ ID NO 6 or SEQ ID NO 7.

The term « immune complex» (also called antigen-antibody complex) describe the result from the interaction between an epitope (antigen) with an antibody directed against this epitope. Said antigen involved in the invention corresponds to the previous described polypeptides consisting of amino acids sequences SEQ ID NO 5 and SEQ ID NO 8.
Detection of said immune complex is carried out with monoclonal or polyclonal antibodies that specifically recognize said antibodies liable to be present in the biological sample of the subject. This recognition is direct.

During the detection procedure, antibodies used for the detection are usually labeled with a marker. Markers used for the labeling of the antibodies are chosen among markers commonly used by the skilled man in the art, and in particular are chosen among radio-isotopic marker, enzymes, fluorescent agents, luminescent agents, magnetic particles... This detection of the formed immune complex is carried out with conventional methods known in the previous art, such as ELISA, immuno-histochemistry and cytochemistry, immunoprecipitation, western blot and any other immunological method. The preferred methods of the invention are ELISA and immunochromatography.

The method of the present invention consists in contacting biological sample of a subject, said biological sample being liable to contain at least one antibody, with polypeptides of the invention, i.e. polypeptides consisting of the amino acids sequences SEQ ID NO 5 and SEQ ID NO : 8.
When said biological sample of the subject contains an antibody liable to be recognize said polypeptides, an immune complex can be formed.
This immune complex is detected by using antibodies that specifically recognize antibodies contained in the biological sample, also called detection antibodies. This immune complex can be eventually quantified, comparing the amount of formed immune complex to amount of control complexes. These control complexes are obtained by using known amount of antibodies that specifically recognize the polypeptides of the invention.
The method of the invention permits therefore to determine if the biologic sample contains antibody directed against the polypeptides described in the invention. The presence of those antibodies allows determining that the individual, where the biologic sample is issuing, is affected by a pathology associated to the EBV virus reactivation.

In a preferred embodiment, the invention discloses a method for *in vitro* and *ex vivo* determination of the presence or amount of at least one antibody, wherein the polypeptide is immobilized on a support, preferably on a micro-titration plate.

In a preferred embodiment, the invention describes a method for determining the presence of antibodies liable to specifically hybridize with polypeptides described above, wherein the method is an ELISA test. Therefore, polypeptides used to capture antibodies contained in the biological sample are fixed in a support. Supports commonly by the killed man in the art include beads and plates. More particularly, polypeptides used in the invention are preferably attached at the bottom of a micro titration plate.

In a preferred embodiment, the detection of the antibody described in the method of the invention corresponds to the Fc fragment of antibody liable to be present in the biological sample of the subject
According to the invention, detection antibodies can interact with the Fc chain of the antibodies contained in the biological sample of the subject.

Also, the disclosure describes an ELISA kit for the *in vitro* and *ex vivo* determination of the EBV reactivation in a subject comprising at least one polypeptide chosen among the group consisting in SEQ ID NO 1, wherein X1 is chosen among an amino acid, with the exception of Proline, or an amino acid sequence comprising at least 2 to 19 amino acids, wherein the last amino acid is not a Proline, X2 and X3, independently from each other, corresponds to an amino acid, with the exception of Proline, wherein the first amino acid is not a Proline, and X4 is chosen among an amino acid, with the exception of Proline, or an amino acid sequence comprising at least 2 to 12 amino acids, or SEQ ID NO 5, or SEQ ID NO 8, and variants and isoforms thereof, immobilized on a support allowing an Enzyme Linked Immunosorbent Assay (ELISA), said support being a common support used in the art such as beads, ELISA plates and micro titration plates.

The ELISA kit disclosed in the invention can also contain materials allowing the detection of the immune complex formation. Then the previously described kit can optionally contain, for example, detection antibodies that recognize the constant chain of immunoglobulins (Fc fragment) of human antibodies. Said detection antibodies are labeled with agent commonly used in the art such as radio-isotopic marker, enzymes, fluorescent agents, magnetic particles...

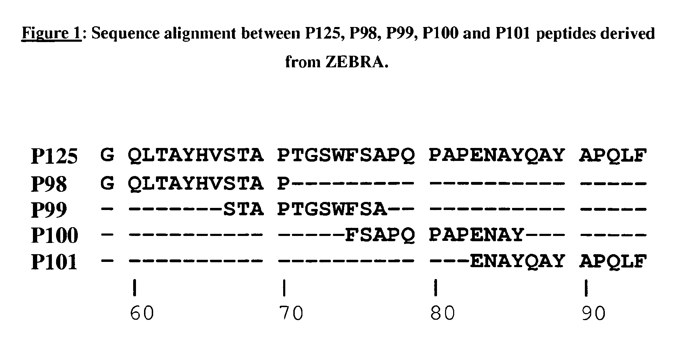

Examples 1 to 5 and figures 1 to 2, which follow, illustrate the invention.

Figure 1 represents the amino acid sequence alignment of P125 ZEBRA polypeptide and the polypeptides thereof P98, P99, P100 and P101. The numbering is based on the numbering of ZEBRA full length protein.

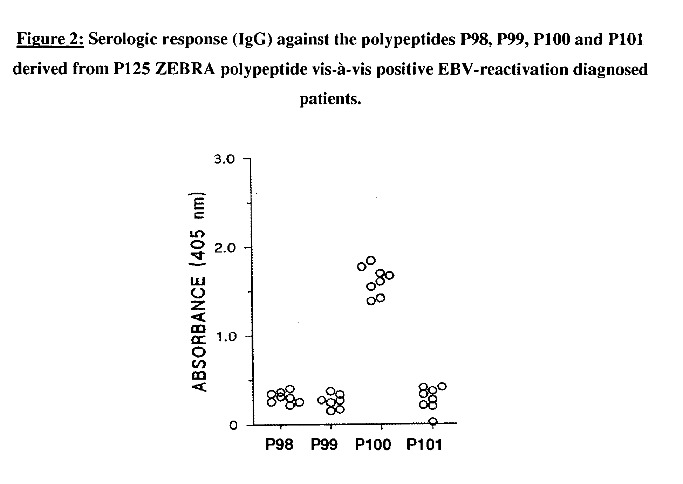

Figure 2 represents the serological reactivity profile of 8 patients with EBV reactivation. The polypeptides P98, P99, P100 and P101, derived from P125 ZEBRA polypeptide have been coated on microtitration plate. The imunoreactivity was determined by measuring the absorbance at 405nm. The X-axis represents respectively P98 (59-70), P99 (67-78), P100 (75-86) and P101 (83-95). The Y-axis represents the relative absorbance in nanometers. Rings represent each patient.

### Example 1: Reactivity of P100 compared to P98, P99 and P101 polypeptides.

### Background:

In previous patent applications, the Inventors have demonstrated that P130 ZEBRA-derived peptide is efficient to determine the EBV primo infection in patients, and P125 ZEBRA-derived peptide is efficient to determine the EBV reactivation in patients afflicted by nasopharyngeal carcinoma.

In order to simplify the immunological test, and to reduce the cost of peptide production, the Inventors analyse the immunogenicity of peptides derived from P125 ZEBRA. 4 polypeptides are generated from P125 ZEBRA peptide, i.e. P98, P99, P100 and P101 polypeptides. The sequence alignment between P98-P101 and P125 is shown in figure 1.

Sera from patients having an activation of EBV lytic cycle are tested by ELISA test, in order to determine the P98-P101 reactivity. These patients are considered to be afflicted by EBV active infection since antibodies against ZEBRA full length protein can be detected in their serum.

### ELISA:

The wells of microtitration plates are coated with the antigen in the following manner:
- The peptides are applied to each well in the proportion of 100ng/100µl (diluted in 50nM carbonate-bicarbonate buffer, pH 9.6).
- The plate is left to incubate overnight at 37°C.
- The plates are washed 3 times with phosphate-0,1% Tween buffer (PBST) pH 7.4.
- The diluted serum is applied to the wells in the proportion of 100µl/well.
- Sera are incubated for 1 hour at 37°C and followed by 3 washes with PBST,
- 100µl of anti-human IgG/peroxidase conjugate are added. IgG/peroxidase conjugates are diluted (in PBST) according to the manufacturer's instructions.
- IgG/peroxidase are incubated for 30 minutes at 37°C and followed by washing with PBST.
- The visualization of peroxidase is carried out using a solution of tetramethylbenzidine (0.5%) and hydrogen peroxide (0.05%) in citrate buffer pH 4 (100µl/well). The reaction duration is for 10 minutes, protected from light.
- The reaction is stopped by adding in each well 1N sulphuric acid solution.
- Reading is carried out in a spectrophotometer and the absorbance is measured at 450 nm (A₄₅₀).
The immunoreactivity of P98-P101 polypeptides is indicated in figure 2.

### Interpretation

The serum from each patient is tested according to the above-described ELISA. All the microtitration wells containing polypeptides P98, P99 or P101 have a low level of reactivity with the 8 tested sera (absorbance 405 nm under 0.5). By contrast, all the tested serum have a high affinity with P100 polypeptide, and present a 7 fold increased absorbance compared to P98-P99 and P101.
Therefore, in P125 ZEBRA polypeptide, only the P100 polypeptide has an efficient reactivity to anti ZEBRA full length protein.

### Example 2: Reactivity of P100 compared to EA, VCA and EBNA EBV-antigens.

### Background:

In the transplantation protocols, patients are treated with immunosuppressive therapies in order to facilitate the engraftment, and to minimize the graft rejection.
However, during these treatments, some patients with a latent EBV infection have an EBV reaction, due to the reduction of the efficiency of the immune system.
So, the EBV reactivation follow-up is indispensable in patients at risk.

The commonly used *in vitro* diagnosis is based on the detection of antibodies directed against VCA, EA and EBNA EBV viral proteins.
A reactivation of the EBV virus (or secondary infection) is defined by the presence of some antibodies directed against:
- VCA (> 1 :320) and
- EA (>1:40).
The presence of these antibodies can be simultaneously detected with the presence (or pre-existence when it has been possible to establish it) of antibodies directed against EBNA (>1:10).

By contrast, a latent infection is defined by :
- the absence of anti-VCA IgM and
- the absence of
   - anti-EA IgG (<1:10),
   - anti-VCA IgG (<1:10) and
   - anti-EBNA IgG (< 1:10).

In order to simplify, and reduce the cost of the EBV reactivation diagnosis, the presence of anti-P100 antibodies is evaluated in patients afflicted, or not, with a pathology associated with an EBV reactivation, and compared to the previously used markers: EBNA, EA and VCA. Moreover, the correlation between the presence of anti-P100 antibodies and the pathologic status of patient is also made.

### Patients:

Sera from a cohort of patients 19 patients (1 to 19) with EBV reactivation are tested, and the presence of antibodies directed against immunogenic peptides of EBV virus is evaluated.
The sera are diluted to 1/100 in PBS (1M NaCl) - 5% fetal calf serum - 0,1% Tween buffer (PBSST).
Detection of anti-P100 antibodies and anti-ZEBRA antibodies (that recognize full-length fusion protein GST-ZEBRA) is made in both sera from:
- transplanted patients with EBV reactivation (patients #1 to #15),
- healthy patients having EBV reactivation (# 16 to # 19), and
- healthy blood donors with latent infection (#20 to #22).
The test is made with the ELISA described in example 1.
Each dilution of serum is tested in duplicate, on the one hand against the peptide antigen, and in the other hand against cups without antigen (control well).
The final value adopted for the absorbance is hence the value resulting from the difference between the mean absorbance of the wells containing the antigen and the mean absorbance of the control wells (ΔA). The following table 1 illustrates the assay of the 22 different sera.

**Table 1: Detection of antibodies directed against ZEBRA derived polypeptide P100, and GST ZEBRA in sera from transplanted patients (with EBV reactivation) (+) indicates a positive reactivity, (-) indicates a negative reactivity.**

| Indirect Immunofluorescence (antibody titer) | | | | ELISA ZEBRA (mean ΔA) | |
|---|---|---|---|---|---|
| Case Nr | Anti-VCA IgG | Anti-EA IgG | Anti-EBNA IgG | Anti-ZEBRA P100 IgG | Anti-GST ZEBRA IgG |
| 1 | 1280 | 20 | 80 | 1,678 (+) | 1,910 (+) |
| 2 | 640 | 40 | 20 | 1,060 (+) | 1,235 (+) |
| 3 | 640 | 20 | 80 | 0,187 (-) | 0,676 (+) |
| 4 | 640 | 80 | 20 | 1,870 (+) | 1,188 (+) |
| 5 | 320 | 40 | 80 | 0,000 (-) | 0,615 (+) |
| 6 | 1280 | 1280 | 160 | 1,432 (+) | 2,047 (+) |
| 7 | 320 | 20 | 10 | 1,560 (+) | 2,143 (+) |
| 8 | 320 | 40 | 20 | 0,880 (+) | 1,782 (+) |
| 9 | 1280 | 320 | 20 | 0,930 (+) | 2,074 (+) |
| 10 | 1280 | 80 | 40 | 0,000 (-) | 2,068 (+) |
| 11 | 640 | 80 | 80 | 0,670 (+) | 1,459 (+) |
| 12 | 320 | 80 | 20 | 0,500 (+) | 1,401 (+) |
| 13 | 1280 | 1280 | 160 | 0,580 (+) | 1,732 (+) |
| 14 | 640 | 40 | 40 | 0,000 (-) | 2,120 (+) |
| 15 | 320 | 20 | 20 | 0,650 (+) | 1,663 (+) |
| 16 | 320 | 40 | 10 | 1,100 (+) | 2,256 (+) |
| 17 | 160 | 20 | 40 | 1,870 (+) | 2,750 (+) |
| 18 | 1280 | 20 | 40 | 0,700 (+) | 1,940 (+) |
| 19 | 160 | 20 | 160 | 1,590 (+) | 2,861 (+) |
| 20 | 20 | <10 | 20 | 0,000 (-) | 0,000 (-) |
| 21 | 160 | <10 | 20 | 0,000 (-) | 0,242 (-) |
| 22 | 80 | <10 | 20 | 0,000 (-) | 0,101 (-) |

### Interpretation:

In the present cohort of 15 transplanted patients with EBV reactivation, 13 patients have significant reactivity using ELISA P100 ZEBRA polypeptides. Only 2 patients (patient #5 and patient #14), although anti VCA, anti EBNA and anti EA antibodies are present that indicates the EBV reactivation, have a negative response to the presence of anti EBV P100 ZEBRA antibodies.
Therefore, the EBV reactivation detection by using P100 ZEBRA polypeptide gives satisfying results with an efficiency of 87%.

The P100 ZEBRA reactivity of sera providing from healthy patients afflicted by EBV reactivation gives similar results, since all the 3 tested patients are positives.
As control, healthy blood donors (with EBV latent infection) have sera that do not react with P100 ZEBRA polypeptide.
Therefore, P100 ZEBRA polypeptides can be easily used to efficiently detect, in patients, the EBV reactivation, whatsoever following or not transplantation. P100 ZEBRA peptide is specific since it does not cross react with sera originating from EBV negative patients or from patient with EBV latent infection.

### Example 3: Kinetic study of anti-P100 antibodies compared to anti VCA, anti-EA and anti-EBNA antibodies.

### Background

The rapid detection of EBV reactivation is an important step to rapidly manage patients.
To date, only the rise in serum of antibodies EA, antibodies VCA and antibodies EBNA is investigated. It is well known that IgG detection follows that of IgM detection.
So the inventors have compared the kinetic of anti EA, anti VCA and anti EBNA antibodies and the anti P100 ZEBRA antibodies as well
The ZEBRA ELISA corresponds to ELISA described in example 1.

2 patients are tested, and correspond to two patients being transplanted. Sera samples are collected at Day 0, corresponding to the day of the beginning of the graft protocol.
After the surgery, sera samples are collected recurrently from 10 to 120 or 210 days.
The presence of anti EA, VCA, EBNA and P100 ZEBRA IgG is evaluated for 120 days (patient #1) or 210 days (patient #2).
Results are indicated in the following table 2.

**Table 2: EBV active infection follow-up of two transplanted patients. (+) indicates a positive reactivity, (-) indicates a negative reactivity.**

| Indirect Immunofluorescence (antibody titer) | | | | ELISA ZEBRA (mean ΔA) | |
|---|---|---|---|---|---|
| Case Nr | Anti-VCA IgG | Anti-EA IgG | Anti-EBNA IgG | Anti-ZEBRA P100 IgG | Anti-ZEBRA P130 IgM |
| Patient #1 | | | | | |
| Day0 | <10 | <10 | <10 | 0,000 (-) | 0,514 (+) |
| **Day10** | **<10** | <10 | <10 | **0,000 (-)** | 0,849 (+) |
| **Day20** | **10** | <10 | <10 | **1,035** (**+)** | 1,479 (+) |
| **Day40** | **80** | 10 | <10 | **0,900 (+)** | 1,453 (+) |
| Day65 | 160 | 20 | <10 | 0,700 (+) | 1,656 (+) |
| Day 120 | 320 | 20 | <10 | 0,538 (+) | 1,470 (+) |

| Patient #2 | | | | | |
|---|---|---|---|---|---|
| Day0 | <10 | <10 | <10 | 0,000 (-) | 0,000 (-) |
| Day10 | <10 | <10 | <10 | 0,000 (-) | 0,000 (-) |
| Day20 | <10 | <10 | <10 | 0,000 (-) | 0,333 (+) |
| Day30 | <10 | <10 | <10 | 0,000 (-) | 0,541 (+) |
| **Day35** | **<10** | <10 | <10 | **0,000 (-)** | 0,721 (+) |
| **Day55** | **10** | <10 | <10 | **0,986 (+)** | 1,520 (+) |
| Day90 | 80 | 10 | <10 | 1,122 (+) | 1,828 (+) |
| Day160 | 1280 | 20 | <10 | 0,692 (+) | 2,025 (+) |
| Day 180 | 1280 | 40 | <10 | 0,580 (+) | 1,900 (+) |
| Day210 | 1280 | 80 | <10 | 0,643 (+) | 1,473 (+) |

### Interpretation:

Patient # 1: This patient has an active EBV infection before the surgery, since at Day 0, IgM against P130 are significantly detected.
Following the surgery, the EBV active infection is detected significantly at Day 20 with P100 ZEBRA polypeptide, but only at Day 40 with VCA polypeptide. Indeed, at Day 10, the anti VCA IgG antibodies titer is not significant to diagnose an EBV active infection, since the value is close by the negative threshold value.
In this case, P100 ZEBRA polypeptide give a significant result about the EBV active infection 10 days before the commonly used detection of anti EA, anti EBNA and anti VCA antibodies.
Patient # 2: This patient is a patient without an active EBV infection before the surgery, since at Day 0, IgM antibodies against P 130 are undetectable.
Following the surgery, the EBV active infection is detected significantly at Day 55 with P100 ZEBRA polypeptide but only at Day 90 with VCA polypeptide. Indeed, at Day 55, the anti VCA antibodies IgG titer is not significant to diagnose an EBV active infection, since the value is close by the negative threshold value.
In this case, P100 ZEBRA polypeptide give a significant result about the EBV active infection 30 days before the commonly used detection of anti EA, anti EBNA and anti VCA antibodies..
These results about the follow-up after a surgery of two transplanted patients demonstrate the precocity of detection of anti P100 ZEBRA antibodies in comparison to antibodies against VCA, EA and EBNA.

### Example 4: Reactivity of P100 compared to EA, VCA, EBNA EBV-antigens and P125.

Sera from a cohort of 5 patients (1 to 5) with EBV reactivation are tested, and the presence of antibodies directed against immunogenic peptides of EBV virus is evaluated.
The sera are diluted to 1/100 in PBS (1M NaCl) - 5% fetal calf serum - 0,1% Tween buffer (PBSST).
Detection of anti-P100 antibodies and anti-P125 antibodies is made in both sera from:
- patients with EBV reactivation (patients #1 to #5), and
- healthy blood donor with latent infection (#6).
Results are represented in the following table 3.

**Table 3: Detection of antibodies directed against ZEBRA derived polypeptide P100, and ZEBRA derived polypeptide P125 in sera from patients (with EBV reactivation). (+) indicates a positive reactivity, (-) indicates a negative reactivity.**

| Indirect Immunofluorescence (antibody titer) | | | | ELISA ZEBRA (mean ΔA) | |
|---|---|---|---|---|---|
| Patient Nr | Anti-VCA IgG | Anti-EA IgG | Anti-EBNA IgG | Anti-ZEBRA P100 IgG | Anti-ZEBRA P125 IgG |
| 1 | 160 | 20 | 40 | 2,456 (+) | 1,870(+) |
| 2 | 320 | 20 | 20 | 1,924 (+) | 1,200 (+) |
| 3 | 160 | 20 | 10 | 1,789 (+) | 1,540 (+) |
| 4 | 1280 | 20 | 40 | 1,400 (+) | 0,700 (+) |
| 5 | 160 | 20 | 160 | 2,670 (+) | 1,590 (+) |
| 6 | 20 | <5 | 20 | 0,150 (-) | (0,000) (-) |

### Interpretation:

In the present cohort of 5 patients with EBV reactivation, all patients have significant reactivity using ELISA P100 ZEBRA polypeptides and P125 ZEBRA.
As control, healthy blood donor (with EBV latent infection) has serum that does not significantly react with P100 ZEBRA polypeptide.
Moreover, P100 ZEBRA polypeptide allows an EBV reactivation detection significantly more important (between 1.3x for patient 1 to 2x for patient 4) than the detection with P125 polypeptide.
P100 ZEBRA peptide is specific since it does not cross react with sera originating from EBV negative patients or from patient with EBV latent infection.

Therefore, P100 is more efficient than P 125 for the detection of EBV reactivation.

### Example 5: ELISA using P100 modified polypeptide (mP100; SEQ ID NO 8).

The ELISA comprising the mP100 polypeptide is the following one:
mP100 is biotinylated in the C terminus.

### Coating:

mP100 polypeptide is diluted at a final concentration 20, 10, 5, 2.5, 1.25, 0.62, 0.31, 0.155, 0.0775 µg/ml in PBS or in a Carbonate/Bicarbonate buffer (K₂CO₃ 140mM, CaHCO₃ 240mM, pH9.5)
100 µl of each dilution are deposited on the wells of plates, said wells being coated with streptavidine. Plates are incubated over-night at 4-8°C, in wet atmosphere.

### Washes:

PBS-tween 0.05% is added in each well, and wells were washed 3 times.
Saturation (blocking)
300 µl/well of PBS- tween 0.05% - BSA 2% are added, and plate is incubated 1h at 37°C

### Test:

Diluted sera are deposited in wells, and plate is incubated 1h30 at 37°C.

As control, 100 µl monoclonal antibody anti-P125 (1.45 mg/ml) diluted 1000 times in PBS-tween 0.05% -BSA(1%)-SVF(5%), is added in wells, in duplicate.

### Washes:

PBS-tween 0.05% is added in each well, and wells were washed 3 times.

### Second antibody (Goat IgG (H+L) anti mouse-Alkaline phosphatase (AP)):

IgG are dilutes 1/2000 in PBS-Tween 0.05%-BSA(1%)-SVF(5%) (0.5%). 100 µl of the dilution is added in each well. Plate is incubated 1h30 at 37°C.
Washes: PBS-tween 0.05% is added in each well, and wells are washed 3 times.

### Revelation (pNPP):

pNPP (5 mg /ml) is prepared in diethanolamine buffer (diethanolamine 0.097M, MgCl₂ 1µM, pH 9.5). 100 µl/well of the diethanolamine solution is used. Development in dark during 30-90 min. Reaction is stopped by addition of 50 µl of NaOH 3N per well.

### DO:

Plates are read at 405 nm and 620 nm (reference).

Similar results, than those obtained in Examples 1-4 with P100 polypeptide, were obtained with P100 modified peptide.

### Bibliography

Epstein MA & Crawford DH, "Gamma herpes viruses: Epstein-Barr Virus", in Topley & Wilsons Microbiology & Microbial infections, Virology Eds BWJ Mahy & V Ter Meulen, 10th edition, Edward Arnold (publishers) 2005.
Klein G. "EBV and the tumor virus context", In Epstein - Barr virus ed by ES Robertson Caister Academic Press, Norfolk UK 2005
Cohen JI. "EBV and the tumor virus context", In Epstein - Barr virus ed by ES Robertson Caister Academic Press, Norfolk UK 2005
Thorley-Lawson DA, Miyashita EM, Khan G. Epstein-Barr virus and the B cell: that's all it takes. Trends Microbiol. 1996 May;4(5):204-8
Speck SH, Chatila T, Flemington E. Reactivation of Epstein-Barr virus: regulation and function of the BZLF1 gene.Trends Microbiol. 1997 Oct;5(10):399-405.
Feederle R, Kost M, Baumann M, Janz A, Drouet E, Hammerschmidt W, Delecluse HJ. The Epstein-Barr virus lytic program is controlled by the co-operative functions of two transactivators. EMBO J. 2000 Jun 15;19(12):3080-9.
Hong GK, Gulley ML, Feng WH, Delecluse HJ, Holley-Guthrie E, Kenney SC. Epstein-Barr virus lytic infection contributes to lymphoproliferative disease in a SCID mouse model. J Virol. 2005 Nov;79(22):13993-4003.
Montone KT, Hodinka RL, Salhany KE, Lavi E, Rostami A, Tomaszewski JE. Identification of Epstein-Barr virus lytic activity in post-transplantation lymphoproliferative disease. Mod Pathol. 1996 Jun;9(6):621-30.
Maréchal V, Meyohas MC, Joab I, Gaha S, Giot JF, Sergeant A, Nicolas JC. Enzyme-linked immunosorbent assay for antibodies to ZEBRA, an Epstein-Barr trans-activator. Res Virol. 1993 Sep-Oct;144(5):397-404.
Joab I, Triki H, de Saint Martin J, Perricaudet M, Nicolas JC. Detection of anti-Epstein-Barr virus trans-activator (ZEBRA) antibodies in sera from patients with human immunodeficiency virus. J Infect Dis. 1991 Jan;163(1):53-6.
Brousset P, Drouet E, Schlaifer D, Icart J, Payen C, Meggetto F, Marchou B, Massip P, Delsol G. Epstein-Barr virus (EBV) replicative gene expression in tumour cells of AIDS-related non-Hodgkin's lymphoma in relation to CD4 cell number and antibody titres to EBV. AIDS. 1994 May;8(5):583-90.
Henle W and Henle G. Clinical spectrum of EBV infection. In the Human herpesviruses: an interdisciplinary perspective. Nahmias, Dowdle and Schinazi eds Elsevier New-York, 1981.
Dardari R, Menezes J, Drouet E, Joab I, Benider A, Bakkali H, Kanouni L, Jouhadi H, Benjaafar N, Gueddari BE, Hassar M, Khyatti M. Analyses of the prognostic significance of the Epstein-Barr virus transactivator ZEBRA protein and diagnostic value of its two synthetic peptides in nasopharyngeal carcinoma. J Clin Virol. 2008 Feb;41(2):96-103. Epub 2007 Nov 19.
Drouet E, Brousset P, Fares F, Icart J, Verniol C, Meggetto F, Schlaifer D, Desmorat-Coat H, Rigal-Huguet F, Niveleau A, Delsol G. High Epstein-Barr virus serum load and elevated titers of anti-ZEBRA antibodies in patients with EBV-harboring tumor cells of Hodgkin's disease J Med Virol. 1999 Apr;57(4):383-9.
Touge C, Agawa H, Sairenji T, Inoue Y.High incidence of elevated antibody titers to Epstein-Barr virus in patients with uveitis. Arch Virol. 2006 May;151(5):895-903.
Tedeschi R, Bloigu A, Ogmundsdottir HM, Marus A, Dillner J, dePaoli P, Gudnadottir M, Koskela P, Pukkala E, Lehtinen T, Lehtinen M. Activation of maternal Epstein-Barr virus infection and risk of acute leukemia in the offspring. Am J Epidemiol. 2007 Jan 15;165(2):134-7.
Dardari R, Hinderer W, Lang D, Benider A, El Gueddari B, Joab I, Benslimane A, Khyatti M. Antibody responses to recombinant Epstein-Barr virus antigens in nasopharyngeal carcinoma patients: complementary test of ZEBRA protein and early antigens p54 and p138. J Clin Microbiol. 2001 Sep;39(9):3164-70.

## Claims

1. Use of the Zebra P100 fragment consisting of the sequence SEQ ID NO 5 or SEQ ID NO 8,
for the in vitro and ex vivo screening of the EBV virus reactivation in a biological sample of a subject afflicted by a pathology associated with EBV infection.

2. Use according to claim 1, wherein pathologies associated with EBV infections are selected from the group comprising: tumors specific to the immunocompromised host, tumor of the immunocompetent host and viral syndrome related to EBV.

3. Method for in vitro and ex vivo determination of the presence or amount of at least one antibody that specifically recognizes the polypeptide consisting of SEQ ID NO 5 or SEQ ID NO 8, said antibody being liable to be present in a biological sample of a subject afflicted by a pathology associated with EBV infection.

4. Method for *in vitro* and *ex vivo* determination of the presence or amount of at least one antibody according to claim 3, said method comprising the following steps:
a. contacting biological sample from said patient with said polypeptide,
b. detecting the formation of an immune complex between said polypeptide and antibody liable to be present in the said biological sample, and
c. optionally quantifying the amount of formed immune complex.

5. Method according to claim 4, wherein polypeptide is immobilized on a support, preferably on a micro-titration plate.

6. Method according to claim 4 or 5, wherein said detection is performed using an antibody able to detect the Fc fragment of said antibody.

7. An ELISA kit for the *in vitro* and *ex vivo* determination of the EBV reactivation in a subject comprising :
- at least one polypeptide consisting of the amino acid sequence SEQ ID NO 5, or SEQ ID NO 8, immobilized in an ELISA plate, and
- optionally, antibodies that recognize Fc fragment of human antibodies.

## Patentansprüche

1. Verwendung des Zebra-P100-Fragments, das aus der Sequenz SEQ ID NO: 5 oder SEQ ID NO: 8 besteht,
für das In-vitro- und Ex-vivo-Screening der EBV-Virus-Reaktivierung in einer biologischen Probe eines Subjekts, das an einer Pathologie leidet, die mit einer EBV-Infektion assoziiert ist.

2. Verwendung nach Anspruch 1, wobei die Pathologien, die mit EBV-Infektionen assoziiert sind, ausgewählt sind aus der Gruppe, umfassend: Tumore, die für den immungeschwächten Wirt spezifisch sind, Tumor des immunkompetenten Wirts und virales Syndrom, das mit EBV in Zusammenhang steht.

3. Verfahren zur In-vitro- und Ex-vivo-Bestimmung der Gegenwart oder der Menge mindestens eines Antikörpers, der das Polypeptid, das aus SEQ ID NO: 5 oder SEQ ID NO 8: besteht, spezifisch erkennt, wobei der Antikörper in einer biologischen Probe eines Subjekts vorhanden sein kann, das an einer Pathologie leidet, die mit EBV-Infektion assoziiert ist.

4. Verfahren zur In-vitro- und Ex-vivo-Bestimmung der Gegenwart oder der Menge mindestens eines Antikörpers nach Anspruch 3, wobei das Verfahren die folgenden Schritte umfasst:
a. Inkontaktbringen einer biologischen Probe des Patienten mit dem Polypeptid,
b. Bestimmen der Bildung eines Immunkomplexes zwischen dem Polypeptid und dem Antikörper, der in der biologischen Probe vorhanden sein kann, und
c. optional, Quantifizieren der Menge des gebildeten Immunkomplexes.

5. Verfahren nach Anspruch 4, wobei das Polypeptid auf einem Träger, bevorzugt auf einer Mikrotiterplatte, immobilisiert ist.

6. Verfahren nach Anspruch 4 oder 5, wobei der Nachweis unter Verwendung eines Antikörpers durchgeführt wird, der in der Lage ist, das Fc-Fragment des Antikörpers nachzuweisen.

7. ELISA-Kit zur *In*-*vitro*- und *Ex*-*vivo*-Bestimmung der EBV-Reaktivierung in einem Subjekt, umfassend:
- mindestens ein Polypeptid, das aus der Aminosäuresequenz SEQ ID NO: 5 oder SEQ ID NO: 8 besteht, das auf einer ELISA-Platte immobilisiert ist, und
- optional Antikörper, die das Fc-Fragment von humanen Antikörpern erkennen.

## Revendications

1. Utilisation du fragment P100 de Zebra consistant en la séquence SEQ ID NO : 5 ou SEQ ID NO : 8,
pour le criblage *in vitro* et *ex vivo* de la réactivation du virus EBV dans un échantillon biologique d'un sujet affecté par une pathologie associée à l'infection par l'EBV.

2. Utilisation selon la revendication 1, où les pathologies associées à l'infection par l'EBV sont choisies dans le groupe comprenant : les tumeurs spécifiques des hôtes immunodéprimés, les tumeurs des hôtes immunocompétents et les syndromes viraux associés à l'EBV.

3. Méthode pour la détermination *in vitro* et *ex vivo* de la présence ou de la quantité d'au moins un anticorps reconnaissant spécifiquement le polypeptide consistant en SEQ ID NO : 5 ou SEQ ID NO : 8, ledit anticorps étant susceptible d'être présent dans un échantillon biologique d'un sujet affecté par une pathologie associée avec l'infection par l'EBV.

4. Méthode pour la détermination *in vitro* et *ex vivo* de la présence ou de la quantité d'au moins un anticorps selon la revendication 3, ladite méthode comprenant les étapes suivantes :
**a-** la mise en contact d'un échantillon biologique issu dudit patient avec ledit polypeptide,
**b-** la détection de la formation d'un complexe immun entre ledit polypeptide et un anticorps susceptible d'être présent dans ledit échantillon biologique, et
**c-** optionnellement la quantification de la quantité de complexe immun formé.

5. Méthode selon la revendication 4, où ledit polypeptide est immobilisé sur un support, préférentiellement sur une plaque de micro-titration.

6. Méthode selon la revendication 4 ou 5, où ladite détection est mise en oeuvre en utilisant un anticorps capable de détecter le fragment Fc dudit anticorps.

7. Kit ELISA pour la détermination *in vitro* et *ex vivo* de la réactivation de l'EBV chez un sujet comprenant :
- au moins un polypeptide consistant en la séquence d'acides aminés SEQ ID NO 5, ou SEQ ID NO : 8, immobilisé sur une plaque ELISA, et
- optionnellement, des anticorps reconnaissant le fragment Fc des anticorps humains.
